# EUROPÄISCHE PATENTSCHRIFT

(11) **EP 0 488 168 B1**
(45) Veröffentlichungstag und Bekanntmachung des Hinweises auf die Patenterteilung: **20.08.1997**
(21) Anmeldenummer: 91120185.3
(22) Anmeldetag: 26.11.1991
(51) Int. Cl.: G01N 33/53

(54) **Verwendung einer Waschlösung mit Komplexbildner für Metallionen in festphasen-immunometrischen Verfahren**
Use of a washing solution containing a complexing agent of metal ions in solid phase immunometric methods
Utilisation d'une solution de lavage contenant un agent complexant d'ions métalliques dans des méthodes immunométriques en phase solide

(30) Priorität: 28.11.1990 DE 4037776
(43) Veröffentlichungstag der Anmeldung: 03.06.1992
(73) Patentinhaber: BEHRINGWERKE Aktiengesellschaft, 35001 Marburg (DE)
(72) Erfinder: Dopatka, Hans-Detlef, Dr., W-3550 Marburg (DE)

(56) Entgegenhaltungen:
- EP-A- 0 328 388
- EP-A- 0 337 785
- EP-A- 0 396 999
- WO-A-86/04610
- US-A- 3 892 841

## Beschreibung

Die Erfindung betrifft eine einen Komplexbildner für Metallionen enthaltende Waschlösung für festphasen-immunometrische Tests und die Verwendung dieser Waschlösung.

Festphasen-immunometrische Tests, beispielsweise der Enzym-Linked-Immunosorbent Assay (ELISA), erfordern bei der Durchführung einen oder mehrere Waschschritte. Hierbei wird die feste Phase mit der Waschlösung gespült, um unspezifisch angelagerte Substanzen, z. B. Immunglobuline, oder überschüssige Reagenzien, z. B. ein Enzymkonjugat, zu entfernen. Geschieht dies in geeigneter Weise, ergibt sich als Testergebnis ein Meßsignal, das der Konzentration des nachgewiesenen Analyten entspricht (Richtigkeit).
Das Testergebnis sollte bei mehrfacher Wiederholung des Tests, bei gleicher Analytkonzentration im Idealfall identisch sein (Reproduzierbarkeit).
Das Waschen wurde in der Vergangenheit meist von Hand mittels Pipetten oder Waschkämmen durchgeführt.

Solche festphasen-immunometrische Tests, können neuerdings auch mittels Instrumenten abgearbeitet werden. Dabei werden zumindest die Waschschritte durch das Instrument durchgeführt.

Instrumente in dem hier verwendeten Sinne sind alle Instrumente, mit deren Hilfe Waschvorgänge in Enzymimmunoassays maschinell durchgeführt werden können, unabhängig davon, ob diese Instrumente in der Lage sind, weitere Schritte der Abarbeitung von ELISA Tests vorzunehmen.

In diesen Systemen weisen die bekannten Waschlösungen, welche z. B. aus detergenzhaltigen Phosphatpuffern im Neutralbereich bestehen, bestimmte Nachteile auf. Bei der Verwendung solcher Instrumente zur Abarbeitung des Waschschrittes erreicht sowohl die Richtigkeit als auch die Reproduzierbarkeit des Meßsignals erst nach einiger Zeit, d. h. nach der Abarbeitung einiger Platten, ein akzeptierbares Maß (BURROWS, P.M. et al., J.Virol.Meth. 8, 207 - 216 (1984)).

In US-A-3892841 wird das Gel der Immunopräzipitationsreaktion einer radial Immunodiffusion mit Komplexbildner gewaschen und mit löslichen Metallsalzen behandelt.

Der vorliegenden Erfindung lag daher die Aufgabe zugrunde, eine Möglichkeit zu finden, durch die bei der Verwendung von Instrumenten zur Abarbeitung von ELISA auch bei sofortigem Gebrauch dieser Geräte korrekte Ergebnisse erzielt werden. Als Maß für die korrekte Testdurchführung gilt ein Meßsignal, das mit der Konzentration des nachgewiesenen Analyten korreliert sowie die Reproduzierbarkeit der erhaltenen Ergebnisse.

Überraschenderweise wurde nun gefunden, daß bei Verwendung einer geeigneten Waschlösung der, unabhängig von der Pufferbasis, dem pH oder anderen Zusätzen Komplexbildner für Metallionen zugesetzt sind, diese Aufgabe gelöst wird.

Gegenstand der Erfindung ist daher die Verwendung eine Waschlösung in heterogenen Enzymimmunoassays, die einen Komplexbildner für Metallionen enthält.

Gegenstand der Erfindung ist ferner ein heterogener Enzymimmunoassay in dem in mindestens einem Waschschritt eine Waschlösung, die einen Komplexbildner für Metallionen enthält, verwendet wird.

Komplexbildner im Sinne dieser Erfindung sind dem Fachmann an sich bekannte Verbindungen, die bevorzugterweise mit höherwertigen Metallionen Komplexe eingehen.

Bevorzugt sind dabei Komplexbildner, die gut biologisch abbaubar sind, wie z. B. Nitrilotriessigsäure (NTA), Isorin-N, N-di-Essigsäure (ISDA), Glukonsäure und Polyglukonsäureacetal, besonders bevorzugt ist NTA.

Der Komplexbildner wird in einer Konzentration von 0,01 mmol/l bis 100 mmol/l zugesetzt. Bevorzugt wird eine Konzentration von 0.1 bis 20 mmol/l, besonders bevorzugt wird eine Konzentration von 1 mmol/l.

Der Komplexbildner kann bereits bekannten Waschlösungen oder Puffern für festphasen-immunometrische Tests zugesetzt werden. Eine davon ist beispielsweise ein 10 mmol/l Phosphatpuffer bestehend aus Na₂HPO₄, KH₂PO₄, NaCl (0,45 % w/v) und 0,1 % (w/v) Tween® 20 mit einem pH von 6,5. Diese Waschlösung wurde als Beispiel erfindungsgemäß mit 1 mmol/l NTA versetzt und diente dazu, die damit im ELISA erzielte Verbesserung des Meßsignals und der Reproduzierbarkeit zu belegen.

Heterogene Enzymimmunoassays sind an sich dem Fachmann bekannt. Sie können zum Nachweis von Antigenen und Antikörpern dienen und additiv, wie z. B. ein Sandwich-Immunoassay, oder kompetitiv sein. Die verschiedenen, möglichen Ausführungsformen sind hinlänglich in der Literatur beschrieben. Bevorzugt im Sinne der Erfindung ist das ELISA-Verfahren.

Markerenzyme für Enzymimmunoassays als solche sind literaturbekannt, bevorzugterweise werden alkalische Phosphatase, β-Galaktosidase und Meerrettich-Peroxidase, besonders bevorzugterweise wird Merrettich-Peroxidase verwendet. Festphasen für heterogene Enzymimmunoassays sind dem Fachmann an sich bekannt, bevorzugterweise werden konkave Formkörper, wie z. B. Röhrchen oder Näpfchen, konvexe Formkörper, wie z. B. Kugeln, Sterne o.ä. und Mikropartikel (Korngröße < 1000 nm) wie z. B. Latexpartikel und magnetisch anziehbare Partikel verwendet. Besonders bevorzugt sind dabei Näpfchen in Form von Mikrotitrationsplatten, Latexpartikel und magnetisch anziehbare Partikel.
Ganz besonders bevorzugt sind Mikrotitrationsplatten.

Materialen für Festphasen sind dem Fachmann bekannt. Soweit nicht, wie z. B. bei Latexpartikeln durch die Art der Festphase bereits festgelegt, wird bevorzugterweise Polystyrol verwendet.

Puffersysteme zur Verwendung in Enzymimmunoassays sind dem Fachmann bekannt. Dem Fachmann ist auch bekannt, daß die Art des jeweils verwendeten Puffersystems vom zu erzielenden pH abhängt.

Detergenzien zur Verwendung in Waschlösungen für heterogene Enzymimmunoassays sind dem Fachmann ebenfalls bekannt (siehe z. B. VOLLER, A. et al. Bull. World Health Organ. 53, 55 - 65 (1976)), bevorzugterweise verwendet werden dabei nichtionische und zwitterionische Detergenzien, besonders bevorzugt sind Polyoxyethylene, ganz besonders bevorzugt ist ^{R}Tween 20.

Neutralproteine zur Verwendung in Enzymimmunoassays sind dem Fachmann bekannt, bevorzugt verwendet werden z. B. Serumalbumine, Gelatine, chemisch modifizierte Gelatine, wie z. B. Polygelin, und Milchproteine, wie z. B. Lactoferrin, besonders bevorzugt sind Human- oder Rinderserumalbumin, Polygelin und Lactoferrin, ganz besonders bevorzugt sind Polygelin und Lactoferrin, letzteres hergestellt gemäß der Patentanmeldung DE 36 38 767 .

Dem Fachmann ist bekannt, daß zur Einstellung einer definierten Osmolarität Neutralsalze, wie z. B. NaCl zu in Enzymimmunoassays verwendeten Lösungen hinzugegeben werden.

Die genannten Stoffe werden zum Gebrauch in wässriger Lösung verwendet; bis zum Gebrauch können sie z. B. in lyophilisierter oder granulierter Form vorliegen, als Trockenmischung oder in flüssiger Form, endverdünnt oder konzentriert.

Eine bevorzugte Ausführung der erfindungsgemäßen Waschlösung setzt sich wie folgt zusammen:
- Puffer: 0 - 100 mmol/l,
bevorzugterweise 10-20 mmol/l
ganz bevorzugterweise 10 mmol/l
- Detergenz: 0 - 1 % (w/v),
bevorzugterweise 0 - 0,2 % (w/v)
ganz bevorzugterweise 0,1 % (w/v)
- Neutralprotein: 0 - 1 % (w/v)
- Komplexbildner: 0,1 - 20 mmol/l,
bevorzugterweise 1-5 mmol/l,
ganz bevorzugterweise 1 mmol/l

Zusammensetzung und Bestandteile herkömmlicher Waschlösungen sind dem Fachmann bekannt.

Die folgenden Beispiele dienen zur Erläuterung der Erfindung und stellen in keiner Weise eine Einschränkung dar.

### Beispiel 1

Als festphasen-immunometrischer Test wurde ein ELISA zum Nachweis von IgM gegen das humane Cytomegalovirus (CMV) gewählt. Als feste Phase dienten Mikrotitrationsplatten aus Polystyrol mit 96 Reaktionsvertiefungen im 8 x 12 Raster.CMV, gezüchtet in Humanen Embryonalen Fibroblasten, und Humane Embryonale Fibroblasten, die nicht mit CMV infiziert waren, wurden nach Krishna et al. (1980), J. Clin. Microbiol. 12, 46 - 51, zu Präparationen aufgearbeitet, die im weiteren als CMV-Antigen bzw. als (negatives) Kontrollantigen bezeichnet werden.

Jede Mikrotitrationsplatte wurde nun durch Einpipettieren von 0,1 ml CMV-Antigen-Lösung in eine Reaktionsvertiefung und von 0,1 ml Kontrollantigen-Lösung in eine benachbarte Reaktionsvertiefung gemäß dem Verfahren der vorgenannten Autoren in der Weise beschichtet, daß Reihen von Reaktionsvertiefungen alternierend beschichtet für CMV-Antigen und Kontrollantigen entstanden. Mehrere Testplatten wurden auf diese Weise in demselben Produktionszyklus hergestellt.

Die Vorverdünnung und das Einpipettieren von je 0,15 ml der Untersuchungsproben erfolgte immer parallel in benachbarte Reaktionsvertiefungen, die einmal mit dem CMV-Antigen und zum anderen mit dem Kontrollantigen beschichtet waren gemäß den Anweisungen von Chou et al (1987), J. Clin. Microbiol. 25, 52 - 55.

Die Abarbeitung des ELISA folgte weitestgehend dem von Ziegelmaier et al (1981), J. Biol. Standard. 9, 23 - 33, beschriebenen Gang von Probeninkubation (1), Konjugatinkubation (2) und Substratinkubation (3), wobei den Reaktionsschritten (2) und (3) ein Waschschritt vorausging.
*) jeweils 3 x 200 µl Waschpuffer innerhalb von 5 min

### Testschema:

### Beschichtung der Festphase mit viralem Antigen

### Waschschritt *)

- Schritt 1:: 150 µl ****)** Testserum in Verdünnungspuffer für Serum und Konjugat (VPSK) werden bei 37°C (für IgG und IgM) inkubiert

**) Volumenangaben jeweils pro Vertiefung

### Waschschritt

- Schritt 2:: 50 µl anti-human IgG x AP Konjugat in VPSK,
60min bei 37°C

### Waschschritt

- Schritt 3:: 100µl p-Nitrophenylphosphat (p-NPP) in Substratpuffer, 45min bei 20-25°C
- Schritt 4:: 50µl 2N-NaOH

### Optische Auswertung

Diese Waschschritte können nicht nur manuell mit einem Waschkamm, sondern auch automatisch mit einem Gerät durch geführt werden, das an ein Vorratsgefäß mit Waschlösung angeschlossen ist. Dem Stand der Technik entsprechen hier z. B. der Ultrawasher II von DYNATECH, der Microplatewasher von Flow Laboratories, der Immuno Washer NK 350 von NUNC, der Easy Washer "EAW plus" von SLT LABINSTRUMENTS oder der Behring ELISA Processor der Behringwerke. In vorgestelltem Beispiel wurde das zuletzt aufgeführte Gerät verwendet.

Abweichend von den Anweisungen von Ziegelmaier et al. wurde nicht ein Anti-Human-IgM-Konjugat mit Alkalischer Phosphatase, sondern eins mit Peroxidase als Markerenzym benutzt. Als Substrat dieses Enzyms wurde Tetramethylbenzidin plus Wasserstoffperoxyd gewählt, welche gemäß den Patentanmeldungen DE 3541978 (28.11.85) und DE 3541979 (18.11.85) hergestellt worden waren; die sich entwickelnde Farbe ist nach 30 min mit 0,1 ml 0,5 N Schwefelsäure gestoppt und bei 450 nm in einem geeigneten Photometer, beispielsweise dem Gerät TitertekR, Multiskan MC, von Flow Laboratories oder dem Behring ELISA Processor der Behringwerke photometrisch vermessen worden. In vorgestelltem Beispiel wurde das zuletzt aufgeführte Gerät verwendet.

Das mit der Untersuchungsprobe in der mit Kontrollantigen beschichteten Reaktionsvertiefung erzielte Meßsignal wurde von dem von derselben Probe in der CMV-Antigen-beschichten Reaktionsvertiegung erzielten Meßsignal subtrahiert. Die Differenz (ΔE) wird als spezifisches Signal (spez. O.D.) bezeichnet und ausschließlich bewertet.

Um den Einfluß der Waschlösung beim instrumentellen Abarbeiten auf die Spezifität und Reproduzierbarkeit des ELISA-Signals zu verdeutlichen, wurde eine einfache Versuchsanordnung gewählt. Eine Testplatte wurde in Mehrfachansätzen (n = 24) nur von zwei Untersuchungsproben belegt. Die Identifikation der einen Probe war PP 1635-3, die der anderen S 81 -184c1. Vier weitere Testplatten wurden in identischer Probenbestückung angesetzt und der ELISA mit allen fünf Testplatten zusammen durchgeführt.

Hierbei wurde darauf geachtet, daß die Reihenfolge der einzelnen Testplatten, eins bis fünf, bei den anfallenden Waschschritten unverändert blieb.

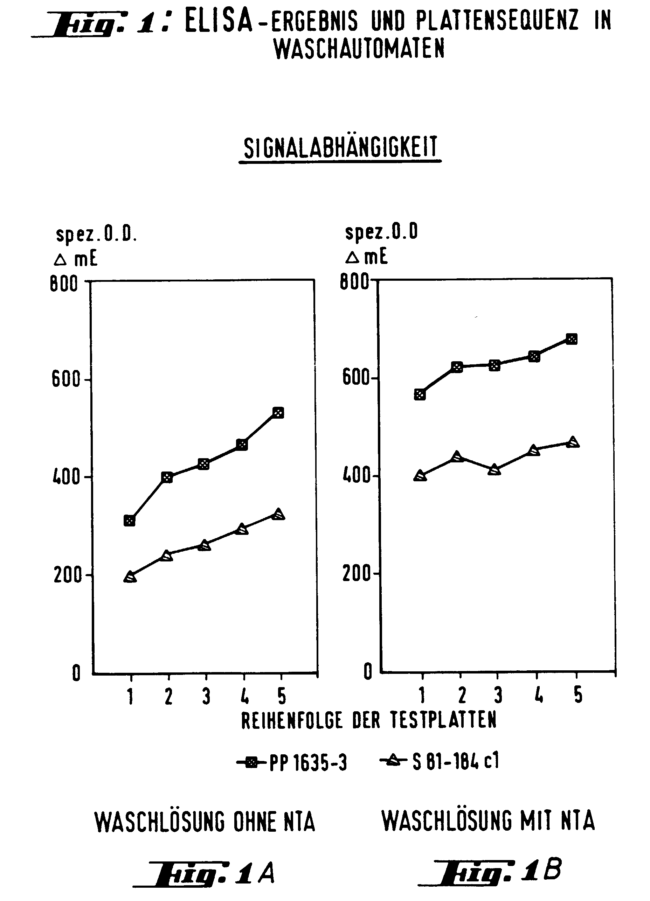

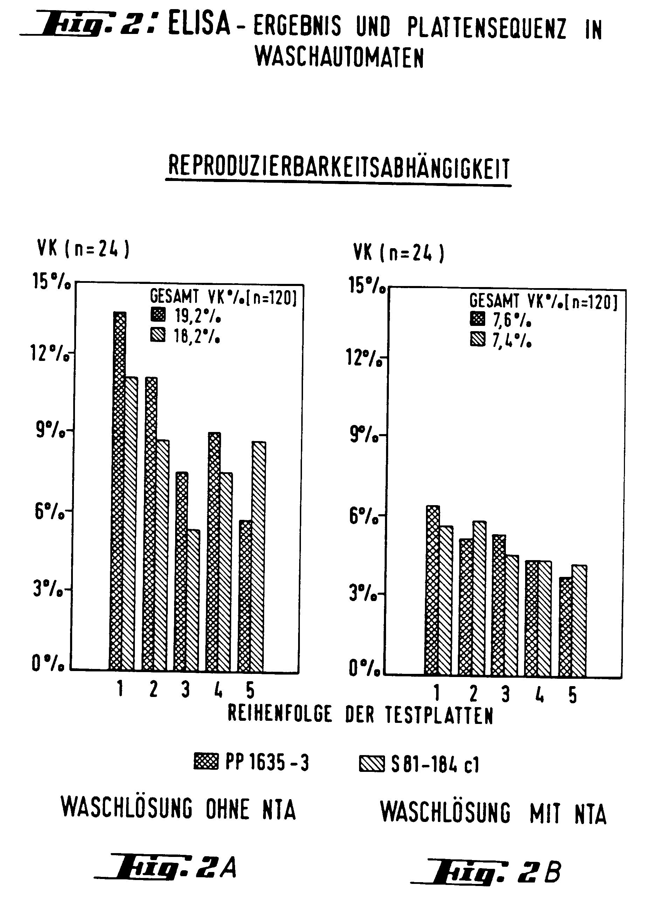

Zunächst wurde, ausgehend vom arithmetischen Mittelwert der spezifischen O.D., die Signalhöhe auf jeder Testplatte in Abhängigkeit von der Waschsequenz dargestellt (Fig 1A). Eindeutig zeigte sich, daß die spezifische O.D. mit der Waschsequenz der Testplatten ansteigt.

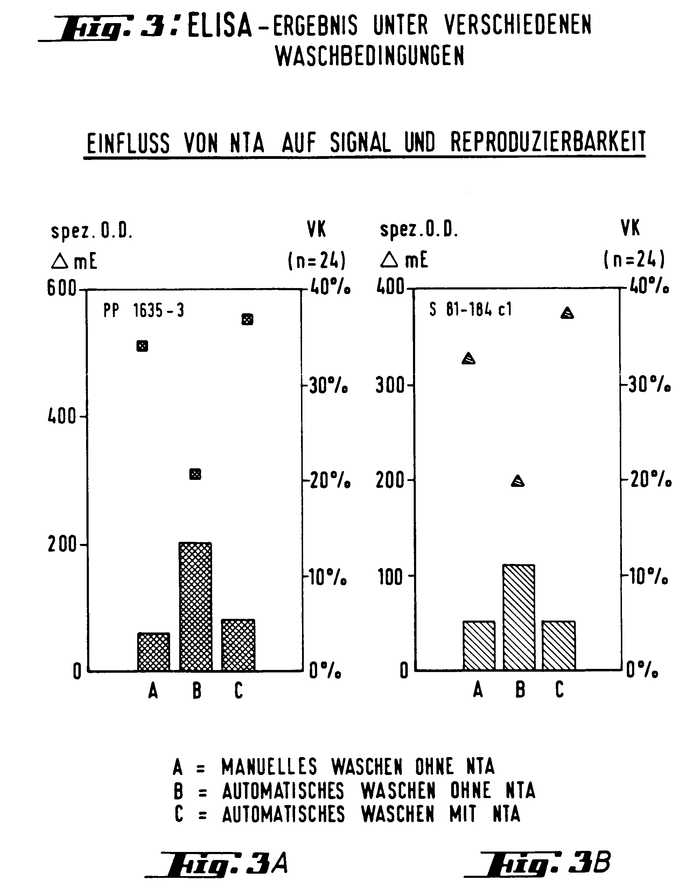

Dann wurde, ausgehend vom Variationskoeffizienten (VK) der spezifischen O.D., die Signalstreuung auf jeder Testplatte in Abhängigkeit von der Waschsequenz dargestellt (Fig. 2A). Eindeutig zeigte sich, daß der VK mit der Waschsequenz der Testplatten niedriger, d. h. besser wird.

Wird der identische Versuchsansatz in der Weise durchgeführt, daß der Waschlösung erfindungsgemäß NTA, z. B. 0,1 mmol/l, zugesetzt wird und diese Waschlösung mit einem Gerät in den Test eingebracht wird, so ergibt sich überraschenderweise ein wesentlich günstigeres ELISA-Ergebnis.

Die Mittelwerte der spezifischen O.D. liegen jetzt unabhängig von der Waschsequenz der Testplatten auf dem der Konzentration des CMV-spezifischen IgM entsprechenden Signalniveau (Fig. 1B). Daneben ist die Signalstreuung unabhängig von der Waschsequenz der Testplatte sehr gering (Fig. 2B).
Wird die auf sämtlichen fünf Testplatten erzielte Meßwert streuung (Gesamt-VK) bewertet, ist die gemäß der Erfindung erreichte Reproduzierbarkeitsverbesserung des ELISA-Ergebnisses noch deutlicher. Bei einer Waschlösung nach dem Stand der Technik beträgt der Gesamt-VK je nach eingesetzter Untersuchungsprobe 18 bis 20 %. Bei einer Waschlösung mit dem erfindungsgemäßen Zusatz von NTA und deren Anwendung beträgt der Gesamt-VK je nach eingesetzter Untersuchungsprobe 7 bis 8 %.

### Beispiel 2

Zur Gegenüberstellung der bekannten Waschverfahren mit der bereits beschriebenen Problematik und der erfindungsgemäßen Waschlösung und deren Verwendung zur Problemlösung dient ein weiterer Versuch. Wieder wurden mit demselben ELISA zum Nachweis von IgM gegen CMV dieselben Untersuchungsproben, nämlich PP 1635-3 und S 81-184c1, in Mehrfachansätzen (n = 24) bestimmt. Diesmal wurden jedoch mehrere unterschiedliche Instrumente zur Durchführung der Waschschritte verwendet. Da der negative Effekt besonders stark die erste abgearbeitete Testplatte betrifft, wurde mittels der unterschiedlichen Instrumente nur jeweils eine einzige Testplatte gewaschen.

Die verwendeten Instrumente können folgendermaßen klassifiziert werden:
- A =: manuelles Waschen mittels 8-fach Multikanalpipette von Flow Laboratories, Waschlösung ohne erfindungsgemäßen Zusatz von NTA.
- B =: automatisches Waschen mittels Behring ELISA Processor, Waschlösung ohne erfindungsgemäßen Zusatz von NTA,
- C =: automatisches Waschen mittels Behring ELISA Processor, Waschlösung mit erfindungsgemäßem Zusatz von NTA.

Der Einfluß der verschiedenen Instrumente in Verbindung mit einer Waschlösung nach dem Stand der Technik bzw. mit einer Waschlösung gemäß Erfindung auf das ELISA-Ergebnis, nämlich spezifisches Meßsignal sowie dessen Reproduzierbarkeit, wurde bewertet.

Dementsprechend wurden der arithmetische Mittelwert und der Variationskoeffizient der spezifischen O.D. ermittelt. Das Ergebnis ist für die vorgenannten Anordnungen A bis C graphisch dargestellt (Anlage 3).

Die Kombination A dient der Kontrolle; hiermit wird gezeigt, welches spezifische Signal der Konzentration des nachgewiesenen Antikörpers entspricht und wie reproduzierbar das Testergebnis sein kann.

Es wird deutlich, daß die Kombination B ein erniedrigtes spezifisches Signal sowie ein Ergebnis schlechter Reproduzierbarkeit erbringt.

In der Kombination C wird dann gezeigt, daß dieser negative Effekt durch die Verwendung der erfindungsgemäßen Waschlösung restlos beseitigt wird; das spezifische Signal erreicht wieder das der Konzentration des nachzuweisenden Antikörpers entsprechende Niveau und die Ergebnisreproduzierbarkeit ist dabei gewährleistet.

## Patentansprüche

1. Verwendung einer Waschlösung, enthaltend einen Komplexbildner für Metallionen in einem heterogenen Enzymimmunoassay.

2. Verwendung gemäß Anspruch 1, dadurch gekennzeichnet, daß der Komplexbildner ein Komplexbildnerfür höherwertige Metallionen ist.

3. Verwendung gemäß Anspruch 1, dadurch gekennzeichnet, daß der Komplexbildner in einer Konzentration von 0,01 - 100 mmol/l eingesetzt wird.

4. Verwendung gemäß Anspruch 3, dadurch gekennzeichnet, daß der Komplexbildner in einer Konzentration von 0,1 - 20 mmol/l eingesetzt wird.

5. Verwendung gemäß Anspruch 4, dadurch gekennzeichnet, daß der Komplexbildner in einer Konzentration von 1 - 5 mmol/l eingesetzt wird.

6. Verwendung gemäß Anspruch 5, dadurch gekennzeichnet, daß der Komplexbildner in einer Konzentration von 1 mmol/l eingesetzt wird.

7. Verwendung gemäß Anspruch 1, dadurch gekennzeichnet, daß die Waschschritte des Enzymimmunoassays mittels eines Gerätes durchgeführt werden.

8. Verwendung gemäß Anspruch 1, dadurch gekennzeichnet, daß das Markierungssystem des Enzymimmunoassays Perioxidase ist.

9. Heterogener Enzymimmunoassay, dadurch gekennzeichnet, daß das Verfahren gemäß Anspruch 1 zur Anwendung kommt.

## Claims

1. The use of a washing solution containing a complexing agent for metal ions in a heterogeneous enzyme immunoassay.

2. The use as claimed in claim 1, wherein the complexing agent is a complexing agent for higher-valent metal ions.

3. The use as claimed in claim 1, wherein the complexing agent is employed in a concentration of 0.01-100 mmol/l.

4. The use as claimed in claim 3, wherein the complexing agent is employed in a concentration of 0.1-20 mmol/l.

5. The use as claimed in claim 4, wherein the complexing agent is employed in a concentration of 1-5 mmol/l.

6. The use as claimed in claim 5, wherein the complexing agent is employed in a concentration of 1 mmol/l.

7. The use as claimed in claim 1, wherein the washing steps in the enzyme immunoassay are carried out by an apparatus.

8. The use as claimed in claim 1, wherein the labeling system in the enzyme immunoassay is peroxidase.

9. A heterogeneous enzyme immunoassay, wherein the method set forth in claim 1 is used.

## Revendications

1. Utilisation d'une solution de lavage, contenant un agent complexant pour ions métalliques, dans un dosage immunoenzymatique hétérogène.

2. Utilisation selon la revendication 1, caractérisée en ce que l'agent complexant est un agents complexant pour ions métalliques à valence élevée.

3. Utilisation selon la revendication 1, caractérisée en ce que l'agent complexant est utilisé à une concentration de 0,01-100 mmoles/l.

4. Utilisation selon la revendication 3, caractérisée en ce que l'agent complexant est utilisé à une concentration de 0,1-20 mmoles/l.

5. Utilisation selon la revendication 4, caractérisée en ce que l'agent complexant est utilisé à une concentration de 1-5 mmoles/l.

6. Utilisation selon la revendication 5, caractérisée en ce que l'agent complexant est utilisé à une concentration de 1 mmole/l.

7. Utilisation selon la revendication 1, caractérisée en ce que l'étape de lavage du dosage immunoenzymatique est effectuée au moyen d'un appareil.

8. Utilisation selon la revendication 1, caractérisée en ce que le système de marquage du dosage immunoenzymatique est la peroxydase.

9. Dosage immunoenzymatique hétérogène, caractérisé en ce que l'on utilise le procédé selon la revendication 1.
